# EUROPEAN PATENT SPECIFICATION

(11) **EP 1 411 996 B1**
(45) Date of publication and mention of the grant of the patent: **15.04.2009**
(21) Application number: 02745043.6
(22) Date of filing: 25.07.2002
(51) Int. Cl.: A61L 9/03, A01M 1/20

(54) **FRAGRANCE DEVICE**
RIECHSTOFFABGABEVORRICHTUNG
DISPOSITIF EMETTANT UN PARFUM

(30) Priority: 01.08.2001 EP 01118487
(43) Date of publication of application: 28.04.2004
(73) Proprietor: Givaudan SA, 1214 Vernier-Genève (CH)
(72) Inventor: BROWN, Colin, Egham, Surrey TW20 8LP (GB); HART, Gerald, Leslie, Surbiton, Surrey KT5 8BD (GB); NAISH, Guy, Edward, Bicester, Oxfordshire OX26 4US (GB)
(74) Representative: Givaudan Patents
(86) International application number: PCT/CH2002/000419
(87) International publication number: WO 2003/011349

(56) References cited:
- EP-A- 0 855 189
- FR-A- 2 529 798
- GB-A- 2 311 223
- US-A- 2 435 756
- US-A- 4 009 384

## Description

This invention is concerned with a device for emitting a fragrance or other volatile substance using a heatsource consisting of a light bulb.

Room air-freshener devices are known that consist of a ring of fragrance-containing material which is engaged on and around a light bulb and which emits fragrance when heated by heat from the light bulb. Typical examples of such devices may be found in United States Patent 2,435,756 and French Demande 2 529 798.

Special heat-resistant materials have been employed in the manufacture of such devices to avoid problems associated with the high temperatures at a light bulb's surface. Typically such materials have included cellulose and asbestos. However, devices made from such materials are brittle and prone to fracture, and the latter material at least is associated with unreasonable health hazards.

More recently it has been suggested to make such devices from special heat-resistant polymers (see US patent 6,120,737). Devices described therein consist of a polymeric ring, spaced apart from the surface of a light bulb; and feet extending radially inward of the ring in the direction of the light bulb and adapted to engage the surface of light bulb in order to support the ring in spaced relationship to the light bulb. Such a device is said to be capable of emitting fragrance to a room for up to 1 month.

However, there are problems associated with this device. Given that the ring is both the carrier for the fragrance and the body providing support for the device, the polymer has to be selected not only for its ability to contain high amounts of fragrance materials and be capable of releasing the fragrance at a desirable rate, it must also have requisite heat-resistant qualities to ensure that its mechanical properties are not compromised at the high temperatures experienced near a light bulb, such that it remains mechanically self-supporting during use. Still further, the device is of relatively complex construction and, as the patentee points out, requires special machinery to insert the feet into the ring immediately after it is formed by injection moulding.

We have now found a fragrance-emitting device employing heat from a light bulb which does not have the disadvantages of the prior art.

Accordingly the invention provides a fragrance-emitting device for releasing fragrances or other volatile materials employing the heat from a light bulb, comprising:
- a hollow container, filled with a fragrance-containing material, and having a central orifice; and
- a base member, the upper surface of which is adapted with electrical connection means, a light bulb received by the connection means, such that the light bulb projects upwardly from said upper surface,
characterised in that the hollow container sits on the base member supported by the upper surface of the base member, and the light bulb is accommodated within the central orifice, the fragrance-containing material is a translucent or transparent gel and the hollow container is formed from translucent or transparent material.

The device is thus a free-standing device wherein the container is supported on a base member, which base member base member may consist of a hollow body, having an upper surface for supporting a light bulb, and defining an internal cavity which may be provided with suitable electronic circuitry for powering the light bulb that is supported on the upper surface of the base member. The light bulb may be supported on the upper surface by any conventional fixing means, e.g. screw fitting or bayonet fitting. Preferably, such a free-standing device is portable, that is, it can be moved around a room into any desired location. In such a preferred embodiment, the power may be supplied by a battery located within the internal cavity, or the light- bulb may be connected to a plug via a flex passing through the base member.

To mimic the flickering effect of a candle and thereby enhance the aesthetics of the device, the electronic circuitry may be adapted to provide, for example an oscillating voltage or provide a random switch between two or more voltages to cause the filament of the light bulb to flicker. Other features may be built into the circuitry to permit, for example, the light to switch on a pre-timed intervals or to respond to other external stimuli, such as a switch that is sensitive to movement or to reduced levels of ambient light.

The hollow container may be of any desirable shape, although typically it may be cylindrical to mimic the more common form of fragranced candles. The central orifice, may also be of any desired shape although, if the wall of the container defining the orifice must abut the light bulb for support, e.g. when the device is to be attached to a light bulb suspended from a ceiling, the orifice must be of the appropriate dimensions for achieving this purpose.

The central orifice may extend completely through the hollow container giving the latter the appearance of a collar or ring. This orifice configuration is necessary for devices designed to be attached to light bulbs suspended from ceilings, but it may be employed in any device of the present invention.
The hollow container may be provided with one or more vent-holes to permit ambient air to contact the fragrance-containing material and to allow egress of volatilised fragrance from the container. Where the viscosity of the fragrance containing material is low such as to permit it to flow out of the vent holes, the vent holes may be covered with a semi-permeable membrane that will permit egress of volatiles but will prevent the unwanted leakage of the fragrance containing material.

The container may be made of any transparent or translucent material that is stable at the high temperatures at or near the surface of a light bulb. By "stable" is meant that the material will not crack or melt, nor will it deform or lose its ability to support its own weight and support the fragrance containing material. Suitable materials may be selected from glass, ceramics and those heat-resistant polymers known in the art.

The hollow container may be supplied separately from the remainder of the device, for example as a refill. As such, the container may be provided with a peelable strip or coating covering the vent holes to prevent evaporation of the fragrance during storage, the coating or strip being adapted to be torn off prior to insertion of the container onto the base member of the device.

The fragrance-containing transparent or translucent gel may be selected from a wide range of polymeric materials known for this purpose. Gels have very high capacity for holding fragrance and/or other volatiles, e.g. they may contain from about 1% up to about 90% or more of fragrance and volatiles, and are capable of releasing these volatiles at a controlled rate over long periods, e.g. 1 month or more. It is a feature of the present invention that these excellent release media may be employed in the present device. Such gels could not be employed in prior art devices as a ring made of gel would at least deform and would probably melt such that the ring could no longer support itself on the light bulb.

Particular gels that may be employed in the present invention are carrageenans, a naturally occurring family of carbohydrates isolated from red seaweed. A typical example is Danagel F 9254 obtainable from FMC Biopolymer, UK. However, other types of gel known for use in air-freshener devices may be employed in a device according to the present invention, for example starches, certain amide polyacrylamides and carboxymethylcellulose. As it is desirable to exploit the aesthetics of a light source, clear gels may be employed, for example gelatine, polysaccharides and other specific polymer systems, for example those disclosed in US 5,780,527.

The device has many advantages. Given that a separate container provides support for the fragrance-containing material, the fragrance-containing material may be selected purely on criteria related to its ability to hold large amounts of fragrance and to release the fragrance at a desirable rate of a prolonged period of time. Furthermore, the container is of relatively simple construction and may be manufactured by simple moulding and filling operations.

For a better understanding of the invention reference is made to the accompanying drawing and descriptive matter that sets forth a preferred embodiment of the invention.

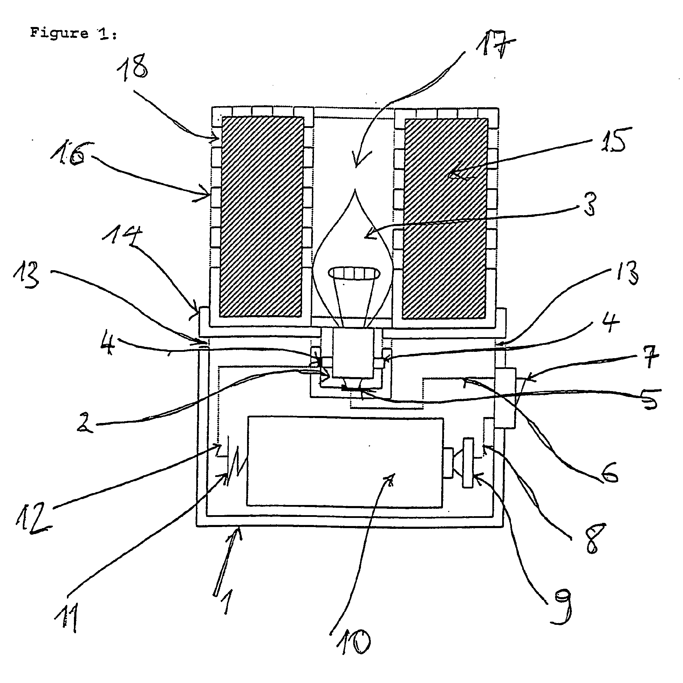

Figure 1 shows a stand-alone portable device. The device comprises a base member (1) the upper surface of which is provided with a recess (2) for receiving a light bulb (3). The recess is equipped with electrical connections (4,5) corresponding to connections on the light bulb. Depending on the type of light bulb fitting, e.g: screw or bayonet, the precise orientation of these connections will vary. The base member has internal wires, the first of which (6) runs from the connector (5) to the on/off switch (7). A second wire (8) then runs to a connector (9) for a battery (10). A second battery connector (11) is attached to a wire (12) which returns to the other electrical connector (4). The base member also has holes (13) to allow air to pass into the cavity and through the recess (2) up past the light bulb (3). On the upper surface of the base member there is provided a raised wall (14) adapted to receive in releasable engagement a gel(15)-containing hollow container (16). The base member is a reusable portion of the device. The hollow container (16) is provided with a central orifice (17) which is adapted to receive the light bulb (3). The vent holes (18) permit egress of fragrance or other volatiles from the container when the device is in operation.

In use, a peelable strip (not shown) protecting the vent-holes (18) would be removed from the hollow container and the container attached to the base member. Secure attachment could be achieved by providing the container wall and the raised wall (14) with, for example co-operating threads, or with other co-operating releasable fixing means. The light bulb is switched on by means of the switch (7) and heat from the light bulb volatilises the fragrance which passes out of the vent holes to freshen the surrounding air. If a transparent container and fragrance-containing material are employed, the light from the light bulb would be visible and would mimic the light from a candle, thereby giving the appearance and ambience of a lighted scented candle. Once the fragrance is exhausted, the hollow container is simply removed from the base member and is replaced by a refill container.

## Claims

1. A fragrance-emitting device for releasing fragrances or other volatile materials employing the heat from a light bulb, comprising:
- a hollow container 16, filled with a fragrance-containing material 15, and having a central orifice 17; and
- a base member 1, the upper surface of which is adapted with electrical connection means 4,5;
- a light bulb 3 received by said connection means, such that the light bulb projects upwardly from said upper surface,
**characterised in that** the hollow container sits on the base member supported by the upper surface of the base member, the light bulb is accommodated within the central orifice, the fragrance containing-material is a translucent or transparent gel and the hollow container is formed from translucent or transparent material.

2. A device according to claim 1 wherein the base member contains therein a battery 10 for supplying power to the light bulb.

3. A device according to claim 2 wherein the light bulb 3 is a flicker-bulb designed to imitate the flickering effect of a candle.

4. A device according to any of the preceding claims wherein the hollow container 16 is provided with vent holes 18 which permit egress of fragrance from inside the hollow container to the surrounding air.

5. Use of a device as defined in any of the preceding claims to emit to surrounding air a fragrance and/or an insecticide.

## Patentansprüche

1. Duftstoffabgabevorrichtung zur Freigabe von Duftstoffen oder anderen flüchtigen Materialien unter Einsatz der Wärme von einer Glühlampe mit
einem hohlen Behälter 16, der mit einem duftstoffenthaltenden Material 15 gefüllt ist und eine mittlere Öffnung 17 hat, und
einem Basiselement 1, dessen obere Fläche mit elektrischen Anschlussmitteln 4, 5 versehen ist,
einer Glühlampe 3, die so von den Anschlussmitteln aufgenommen ist, dass die Glühlampe von der oberen Fläche nach oben vorragt,
**dadurch gekennzeichnet, dass** der hohle Behälter auf dem Basiselement sitzt und von der oberen Fläche des Basiselements gestützt wird, die Glühlampe in der mittleren Öffnung untergebracht ist, das duftstoffhaltige Material ein durchscheinendes oder transparentes Gel ist und der hohle Behälter aus durchscheinendem oder transparentem Material gebildet ist.

2. Vorrichtung nach Anspruch 1, wobei das Basiselement eine Batterie 10 zur Speisung der Glühlampe mit Energie enthält.

3. Vorrichtung nach Anspruch 2, wobei die Glühlampe 3 eine Flackerlampe ist, die dazu ausgelegt ist, die Flackerwirkung einer Kerze nachzuahmen.

4. Vorrichtung nach einem der vorhergehenden Ansprüche, wobei der hohle Behälter 16 mit Belüftungslöchern 18 versehen ist, die das Austreten von Duftstoff aus dem Innern des hohlen Behälters an die Umgebungsluft gestatten.

5. Verwendung einer Vorrichtung nach einem der vorhergehenden Ansprüche, zur Abgabe eines Duftstoffs und/oder eines Insektizids an die Umgebungsluft.

## Revendications

1. Dispositif émettant un parfum pour libérer des parfums ou d'autres substances volatiles employant la chaleur d'une ampoule, comprenant :
- un récipient creux 16, rempli d'une substance contenant le parfum 15, et ayant un orifice central 17 ; et
- un organe de base 1, dont la surface supérieure est pourvue de moyens de connexion électriques 4, 5 ;
- une ampoule 3 reçue par lesdits moyens de connexion, de telle sorte que l'ampoule fasse saillie vers le haut depuis ladite surface supérieure,
**caractérisé en ce que** le récipient creux repose sur l'organe de base de manière supportée par la surface supérieure de l'organe de base, l'ampoule est reçue dans l'orifice central, la substance contenant le parfum est un gel translucide ou transparent et le récipient creux est formé à partir d'un matériau translucide ou transparent.

2. Dispositif selon la revendication 1, dans lequel l'organe de base contient une pile 10 pour alimenter l'ampoule en puissance.

3. Dispositif selon la revendication 2, dans lequel l'ampoule 3 est une ampoule scintillante conçue pour imiter l'effet de scintillement d'une bougie.

4. Dispositif selon l'une quelconque des revendications précédentes, dans lequel le récipient creux 16 est pourvu de trous de ventilation 18 qui permettent la sortie de parfum depuis l'intérieur du récipient creux vers l'air ambiant.

5. Utilisation d'un dispositif selon l'une quelconque des revendications précédentes pour émettre vers l'air ambiant un parfum et/ou un insecticide.
